(19) Europäisches Patentamt
European Patent Office
Office européen des brevets

(11) Veröffentlichungsnummer: **0 116 175**
**B1**

(12) **EUROPÄISCHE PATENTSCHRIFT**

(45) Veröffentlichungstag der Patentschrift: **15.02.89**

(51) Int. Cl.⁴: **A 61 K 31/53,** A 61 K 9/08, A 61 K 47/00

(21) Anmeldenummer: **83113224.6**

(22) Anmeldetag: **30.12.83**

(54) **Coccidiosemittel.**

(30) Priorität: **12.01.83 DE 3300793**

(43) Veröffentlichungstag der Anmeldung: **22.08.84 Patentblatt 84/34**

(45) Bekanntmachung des Hinweises auf die Patenterteilung: **15.02.89 Patentblatt 89/7**

(84) Benannte Vertragsstaaten: **AT BE CH DE FR GB IT LI LU NL SE**

(56) Entgegenhaltungen:
**EP-A-0 061 142**
**FR-A-1 476 867**
**FR-A-2 388 559**

(73) Patentinhaber: **BAYER AG, Konzernverwaltung RP Patentabteilung, D-5090 Leverkusen 1 Bayerwerk (DE)**

(72) Erfinder: **Voege, Herbert, Dr., Martin- Buber-Strasse 41, D-5090 Leverkusen 3 (DE)**

**EP 0 116 175 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die Erfindung betrifft wassermischbare Lösungen von 1-[3-Methyl-4-(4'-trifluormethylthiophenoxy)-phenyl]-3-methyl-1,3,5-triazin-2,4,6-(1H, 3H, 5H)-trion und deren Verwendung bei der Bekämpfung von Coccidiosen.

Die obengenannte Verbindung, deren chemische Strukturformel im folgenden wiedergegeben wird,

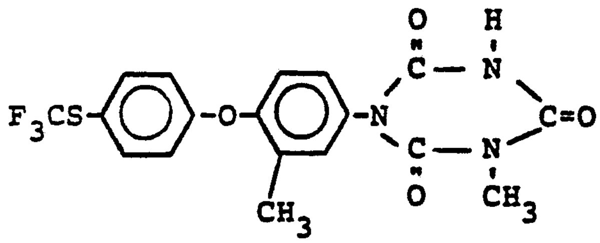

(I)

ist als Wirkstoff gegen Coccidiose-Erkrankungen und ähnlichen Erkrankungen von Tieren bekannt. Bisher wurde dieser Wirkstoff in kristalliner gemahlener Form zur Behandlung dem Futter der Tiere zugesetzt. Für eine Behandlung über das Trinkwasser, für die vielerorts ein großes Bedürfnis besteht, konnte aber bisher keine brauchbare Anwendungsform gefunden werden. Um eine sichere Anwendung über das Trinkwasser zu sichern, muß ein solcher Wirkstoff etwa 24 Stunden im Wasser homogen verteilt vorliegen. Der Wirkstoff löst sich nur zu etwa $\leqq$ 5 ppm, d.h. nicht genügend in Wasser auf. Als Suspension eingebracht sedimentiert er in diesem Zeitraum in der Anwendungskonzentration.

Die Anwendungs-Konzentration kann je nach Schwere der Erkrankung zwischen 5 - 500 ppm liegen, meistens jedoch zwischen 20 - 200 ppm.

Der Wirkstoff der Formel (I) löst sich in verschiedenen organischen Lösungsmitteln gut, so z. B. in Aceton, Milchsäureethylester oder N-Methylpyrrolidon. Beim Verdünnen einer solchen Lösung im Trinkwasser für Tiere auf die Anwendungs-Konzentration fällt der Wirkstoff aber sofort oder nach kurzer Zeit aus. Auch eine Solubilisierung mit Solubilisatören wie polyoxyethyliertem Rizinusöl oder Polyoxethylen-Sorbitan-Fettsäureestern führt nicht zum Erfolg. Das Ausfällen des Wirkstoffes wird zwar um einige Stunden verzögert, nich aber während 24 Stunden.

Überraschenderweise wurde nun gefunden, daß wassermischbare Lösungen des Wirkstoffs der Formel (I), die ein oder mehrere polare Lösungsmittel enthalten und alkalisch reagieren, im Trinkwasser der Tiere auf die Anwendungskonzentrationen verdünnt werden können und über 24 Stunden nicht ausfallen.

Gegenstand der Erfindung sind demgemäß wassermischbare Lösungen von 1-[3-Methyl-4-(4'-trifluormethylthiophenoxy)-phenyl]-3-methyl-1,3,5-triazin-2,4,6-(1H, 3H, 5H)-trion, die dadurch gekennzeichnet sind, daß sie ein oder mehrere polare Lösungsmittel enthalten und alkalisch reagieren.

Zur Herstellung der erfindungsgemäßen Lösungen wird der Wirkstoff in einem polaren, wasserlöslichen Lösungsmittel gelöst, welches entweder alkalisch reagiert oder dem eine alkalische wasserlösliche Substanz zugefügt wird. Letztere wird vorteilhafter ebenfalls im Lösungsmittel gelöst, kann aber auch in dem Lösungsmittel suspendiert sein und sich erst im Trinkwasser lösen. Dabei soll das Trinkwasser nach Zusatz der Wirkstofflösung einen pH-Wert von mehr als 7, vorzugsweise aber einen pH-Wert größer als pH 8 haben.

Die Lösung des Wirkstoff-Konzentrats sollte einen pH von 11 nicht überschreiten und einen pH von 8 nicht unterschreiten.

Die Konzentration des Wirkstoffes kann im Bereich von 0,5 - 50 % liegen, vorzusweise aber in einem Bereich von 1 - 25 %.

Als Lösungsmittel kommen alle wasserlöslichen Lösungsmittel in Betracht, in demen der Wirkstoffin genügender Konzentration löslich ist und die physiologisch unbedenklich sind.

Dies sind aus der Reihe der Alkohole ein- und mehrwertige wie Ethylalkohol, Isopropylalkohol, Benzylalkohol, Glycerin, Propylenglykol, Polyethylenglykole, Poly(oxyethylen)-poly(oxypropylen)-Polymere, basische Alkohole wie z. B. Mono-, Di- und Triethanolamin.

Außerdem sind geeignet Ketone z. B. Aceton oder Methylethylketon und aus der Reihe der Ester z. B. Milchsäureethylester. Andere Lösungsmittel wie N-Methylpyrrolidon, Dimethylacetamid, Dimethylformamid können ebenfalls eingesetzt werden.

Als Basen zur Einstellung des alkalischen pH-Wertes sind vorzugsweise organische Basen einzusetzen, z. B. basische Aminosäuren wie L- bzw. D,L-Arginin, L- bzw. D, L-Lysin, aber auch Cholin, Methylglucosamin, Glucosamin, 2-Amino-2-hydroxymethylpropandiol-(1,3). Auch Diamine sind hier geeignet z. B. bildet N, N, N', N'-tetrakis-(2-hydroxypropyl)-ethylendiamin oder Polyether-Tetrol auf der Basis Ethylendiamin (M.G. 480 - 420, OH-Index 432 - 467) ebenfalls klare Lösungen im angegebenen H-Bereich aus. Auch anorganische Basen, können eingesetzt werden, z. B. Ammoniak oder Natriumcarbonat - gegebenenfalls unter Zugabe von Wasser.

Werden Stoffe und Mittel, die zur Prophylaxe und/oder Medikation geeignet sind, gleichzeitig mit Coccidienmitteln an die Tiere verabreicht, so können sie zusätzlich in die Formulierung miteingebracht werden um als basische Komponente der Formulierung zu dienen, z. B. Aminoglykosid-Antibiotika, wie Streptomycin, Gentamicin, Sisomicin, Eomicin, oder Makrolid-Antibiotika wie Tylosin oder Kitasamycin oder Natriumsalze von Sulfonamiden.

Substanzen, die sonst als Emulgatoren oder Solubilisatoren verwendet werden und in Wasser kolloidal

löslich sind, können in diesem Falle wie polare Lösungsmittel eingesetzt werden, sofern ihnen noch ein basischer Hilfsstoff zugemischt wird.

Wie eingangs erwähnt wurde, gelingt es ohne Zusatz von alkalisch wirkenden Stoffen nicht, den Wirkstoff durch Solubilisieren längere Zeit in der Endkonzentration in Lösung zu halten. Solche Emulgatoren können aber den oben erwähnten, beanspruchten Lösungen zugesetzt werden, um z. B. die Verteilung in Wasser zu erleichtern oder suspendierte Hilfsstoffe zu benetzen. Insbesondere polyoxyethylierte Substanzen kommen hier in Frage wie z. B. polyoxyethyliertes Rizinusöl, Polyethylenglykol-Sorbitan-Monooleat, Polyethylenglykol-Nonylphenyl, Polyethylenglykolstearat, oder Polyethylenglykolether. Basische Derivate wie Polyethylenglykol-Alkylamine sind hier besonders vorteilhaft.

Lösungen bzw. Suspensionen der oben geschilderten Art können auch 0,1 bis 20 Gew.-%, vorzugsweise 0,1-10 Gew.-% anderer Formulierhilfsstoffe, wie Antioxydantien, andere Tenside, Suspensionsstabilisatoren und Verdickungsmittel wie z. B. Methylcellulose, kolloidale Kieselsäure und anderes enthalten. Der Zusatz von Farbe, Aroma und Aufbaustoffe zur Tierernährung ist ebenfalls möglich. Auch Säuren, die mit der vorgelegten Base zusammen ein Puffersystem bilden oder den pH der Lösung reduzieren, sind hier zu nennen.

Die folgenden Beispiele sollen die Art der Erfindung aufzeigen ohne sie dadurch einzugrenzen.

Zur Herstellung der erfindungsgemäßen Lösungen werden die Substanzen in einen Behälter mit Rührwerk eingewogen und dann unter Erwärmen solange gerührt, bis eine klare Lösung entstanden ist. In den aufgeführten Beispielen ist der Wirkstoff über einen Monat bei 50°C stabil. Werden die Lösungen aus den Beispielen 1 : 1000 mit Wasser verdünnt, liegt der pH des Wassers in dem oben beschriebenen Bereich von größer 8.

**Beispiel 1**

2,5 g Wirkstoff werden zu 100 ml in Triethanolamin unter Erwärmen gelöst.
Die klare Lösung hat einen pH-Wert von 10,2.

**Beispiel 2**

2,5 g Wirkstoff und 12,5 g Milchsäure
werden zu 100 ml in Triethanolamin unter Erwärmen und Rühren gelöst.
Der pH der Lösung beträgt 8,3.

**Beispiel 3**

10,0 g Wirkstoff wird zu 100 ml in Monoethanolamin gelöst.
Die klare Lösung hat einen pH-Wert von 11.

**Beispiel 4**

| | | |
|---|---|---|
| Wirkstoff | | 5,0 g |
| Propylenglykol | | 50,0 g |
| Natriumcarbonat | | 5,0 g |
| Wasser | ad | 100 ml |

pH der Lösung 9,9.

**Beispiel 5**

5,0 g Wirkstoff
25,0 g D,L-Lysin Base
ad 100 ml Polyethylenglykol 400
pH der Lösung 9,8

**Beispiel 6**

25,0 g Wirkstoff
10,0 g Monoethanolamin
ad 100 ml N-Methylpyrrolidon
pH der Lösung 10,8.

## Patentansprüche

1. Wassermischbare Lösungen von 1-[3-Methyl-4-(4'-trifluormethylthiophenoxy)-phenyl]-3-methyl-1,3,5-triazin-2,4,6-(1H, 3H, 5H)-trion, dadurch gekennzeichnet, daß sie ein oder mehrere polare Lösungsmittel enthalten und alkalisch reagieren.
2. Wassermischbare Lösungen nach Anspruch 1, dadurch gekennzeichnet, daß ihr pH-Wert 8 bis 11 beträgt.
3. Wassermischbare Lösungen nach Anspruch 1, dadurch gekennzeichnet, daß als Lösungsmittel ein alkalisch reagierendes polares Lösungsmittel eingesetzt wird.
4. Wassermischbare Lösungen nach Anspruch 3, dadurch gekennzeichnet, daß als Lösungsmittel Ethanolamin eingesetzt wird.
5. Wassermischbare Lösungen nach Anspruch 1, dadurch gekennzeichnet, daß sie 1-[3-Methyl-4-(4'-triflourmethyl-thiophenoxy)-phenyl]-3-methyl-1,3,5-triazin-2,4,6 (1H, 3H, 5H)-trion in Konzentrationen von 0,5 - 50 Gew.-% enthalten.
6. Wassermischbare Lösungen nach Anspruch 1, dadurch gekennzeichnet, daß sie den Wirkstdoff in einer Konzentration von 1 - 25 Gew.-% enthalten.
7. Wassermischbare Lösungen nach Anspruch 1, derart durch alkalisch reagierende Zusätze eingestellt, daß sie nach Verdünnung mit Wasser auf Anwendungskonzentration einen pH-Wert von 8 bis 11 aufweisen.
8. Verfahren zur Herstellung wassermischbarer Lösungen nach Anspruch 1, dadurch gekennzeichnet, daß man 1-[3-Methyl-4-(4'-trifluormethylthiophenoxy)-phenyl]-3-methyl-1,3,5-triazin-2,4,6 (1H, 3H, 5H)-trion in einem polaren, gegebenenfalls alkalisch reagierenden, wassermischbaren Lösungsmittel löst und die Lösung durch gegebenenfalls Lösen oder Suspendieren alkalisch reagierender Zusätze auf einen pH-Wert von 8 bis 11 einstellt.

## Claims

1. Water-miscible solutions of 1-[-3-methyl-4-(4'-trifluoromethylthiophenoxy)phenyl]-3-methyl-1,3,5-triazine-2,4,6(1H,3H,5H)-trione, characterized in that they contain one or more polar solvents and have an alkaline reaction.
2. Water-miscible solutions according to Claim 1, characterized in that their pH is 8 to 11.
3. Water-miscible solutions according to Claim 1, characterized in that the solvent employed is a polar solvent having an alkaline reaction.
4. Water-miscible solutions according to Claim 3, characterized in that ethanolamine is employed as the solvent.
5. Water-miscible solutions according to Claim 1, characterized in that they contain 1-[3-methyl-4-(4'-triflouromethylthiophenoxy)phenyl]-3-methyl-1,3,5-triazine-2,4,6(1H,3H,5H)-trione in concentrations of 0.5 - 50 % by weight.
6. Water-miscible solutions according to Claim 1, characterized in that they contain the active compound in a concentration of 1 - 25 % by weight.
7. Water-miscible solutions according to Claim 1, adjusted by additives having alkaline reactions such that, after dilution with vater to the concentration for administration, they have a pH of 8 to 11.
8. Process for the preparation of water-miscible solutions according to Claim 1, characterized in that 1-[3-methyl-4-(4'-triflouromethylthiophenoxy)phenyl]-3 methyl-1,3,5-triazine-2,4,6(1H,3H,5H)-trione is dissolved in a polar, water-miscible solvent which optionally has an alkaline reaction, and the solution is adjusted to a pH of 8 to 11 by, where appropriate, dissolving or suspending additives having an alkaline reaction.

## Revendications

1. Solutions miscibles à l'eau de 1-[3-méthyl-4-(4'-trifluorométhyl-thiophénoxy)-phényl]-3-méthyl-1,3,5-triazine-2,4,6-(1H, 3H, 5H)-trione, caractérisées en ce qu'elles contiennent un ou plusieurs solvants polaires et réagissent de manière alcaline.
2. Solutions miscibles à l'eau suivant la revendication 1, caractérisées en ce que leur pH se situe entre 8 et 11.
3. Solutions miscibles à l'eau suivant la revendication 1, caractérisées en ce que l'on utilise un solvant polaire

à réaction alcaline en tant que solvant.

4. Solutions miscibles à l'eau suivant la revendication 3, caractérisées en ce que l'on utilise l'éthanolamine en tant que solvant.

5. Solutions miscibles à l'eau suivant la revendication 1, caractérisées en ce qu'elles contiennent la 1-[3-méthyl-4-(4'-trifluorométhyl-thiophénoxy)-phényl]-3-méthyl-1,3,5-triazine-2,4,6-(1H, 3H, 5H)-trione dans des concentrations de 0,5 à 50 % en poids.

6. Solutions miscibles à l'eau suivant la revendication 1, caractérisées en ce qu'elles contiennent la substance active dans une concentration de 1 à 25 % en poids.

7. Solutions miscibles à l'eau suivant la revendication 1, ajustées par des additifs à réaction alcaline de telle manière que, après la dilution dans l'eau à la concentration utile, elles présentent un pH dans l'intervalle de 8 à 11.

8. Procédé de fabrication de solutions miscibles à l'eau suivant la revendication 1, caractérisé en ce que l'on dissout la 1-[3-méthyl-4-(4'-trifluorométhyl-thiophénoxy)-phényl]-3-méthyl-1,3,5-triazine-2,4,6(1H, 3H, 5H)-trione dans un solvant polaire miscible à l'eau éventuellement à réaction alcaline, et en ce qu'on ajuste le pH de la solution, éventuellement par dissolution ou suspension d'additifs à réaction alcaline, à une valeur de 8 à 11.